# EUROPEAN PATENT APPLICATION

(11) **EP 4 640 676 A1**
(43) Date of publication of application: **29.10.2025**
(21) Application number: 23906011.4
(22) Date of filing: 20.12.2023
(51) Int. Cl.: C07D 401/04, A61K 31/416, A61K 31/506, A61P 9/00

(54) **CRYSTAL FORM OF FLUORINE-SUBSTITUTED INDAZOLE COMPOUND AND USE THEREOF**

(30) Priority: 21.12.2022 CN 202211648031
(71) Applicant: Sunshine Lake Pharma Co., Ltd., Dongguan, Guangdong 523000 (CN)
(72) Inventor: CHEN, Liang, Dongguan, Guangdong 523871 (CN); SHAN, Yuefeng, Dongguan, Guangdong 523871 (CN); WANG, Xiaojun, Dongguan, Guangdong 523871 (CN); ZUO, Yinglin, Dongguan, Guangdong 523871 (CN)
(74) Representative: Hoffmann Eitle
(86) International application number: PCT/CN2023/140301
(87) International publication number: WO 2024/131850

(57) **Abstract**

The present invention relates to a crystal form of a fluorine-substituted indazole compound and a use thereof. The present invention also relates to a pharmaceutical composition comprising the crystal form. Specifically, the crystal form or the pharmaceutical composition can be used for treating and preventing cardiovascular diseases or conditions.

## Description

### FIELD OF THE INVENTION

The present invention belongs to the field of pharmaceutical technology and relates to crystal form of fluorine-substituted indazole compound and use thereof. Specifically, the invention pertains to the crystal form of methyl (4,6-diamino-2-(7-fluoro-1-(2-fluorobenzyl)-1H-indazol-3-yl)pyrimidin-5-yl)carbamate and use thereof, and further relates to the pharmaceutical composition comprising the crystal form and use thereof.

### BACKGROUND

Soluble guanylate cyclase (sGC) is widely present in the cytosol of mammalian cells, with relatively high concentrations in the lungs and brain. It is a key signal transduction enzyme in the nitric oxide (NO)-sGC-cyclic guanosine monophosphate (cGMP) signaling pathway. When activated in vivo, sGC catalyzes the conversion of GTP to cGMP. cGMP is an important secondary messenger molecule that triggers a series of cascade reactions downstream by activating various downstream effectors, such as phosphodiesterase (PDE), cyclic nucleotide-gated ion channel (CNG), and protein kinase G (PKG). It plays vital physiological roles in the gastrointestinal, blood circulatory and nervous systems, including promoting vascular and smooth muscle relaxation, inhibiting platelet aggregation, vascular remodeling, apoptosis, and inflammation, as well as participating in neurotransmission.

sGC stimulators have a dual mechanism of action on sGC in vivo. When NO concentration is low, they can directly activate sGC; when NO reaches a certain level, they can synergize with NO to activate sGC, thereby catalyzing the conversion of substrate guanosine triphosphate (GTP) into the second messenger molecule cyclic guanosine monophosphate (cGMP), so as to subsequently participates in regulating many important physiological processes, such as promoting vasodilation and smooth muscle relaxation, and inhibiting platelet aggregation, vascular remodeling, etc. sGC stimulators stimulate sGC to mediate other signaling pathways such as TGF-β, thereby exerting anti-fibrotic and anti-tumor effects. Therefore, the NO/sGC/cGMP pathway has become an effective therapeutic target for various cardiovascular diseases, including pulmonary hypertension, acute heart failure, angina pectoris and vascular remodeling induced by myocardial infarction.

Experimental and clinical evidence shows that sGC stimulators can be used to treat cardiovascular diseases, including systemic and pulmonary hypertension, heart failure, angina pectoris, stroke, thrombosis and other thromboembolic disease, peripheral arterial disease, hepatic, pulmonary or kidney fibrosis, and atherosclerosis; they can also be used to treat lipid-related conditions, such as dyslipidemia, hypercholesterolemia, hypertriglyceridemia, sitosterolemia, fatty liver disease and hepatitis.

Patent application WO2018188590A1 discloses a class of fluorine-substituted indazole compounds as soluble guanylate cyclase (sGC) stimulators, specifically the compound methyl (4,6-diamino-2-(7-fluoro-1-(2-fluorobenzyl)-1H-indazol-3-yl)pyrimidin-5-yl)carbamate (Example 10), its synthesis method and its uses.

Drug polymorphism is a common phenomenon in drug development and an important factor affecting the quality of drugs. Different crystal forms of the same drug may have significant differences in appearance, solubility, melting point, dissolution, bioavailability, etc., and may also have different effects on the stability, bioavailability and efficacy of the drug. Therefore, the issue of drug polymorphism should be comprehensively considered in the research and development of a drug.

### SUMMARY

Through extensive research, the inventors have discovered the crystal forms of the compound methyl (4,6-diamino-2-(7-fluoro-1-(2-fluorobenzyl)-1H-indazol-3-yl)pyrimidin-5-yl)carbamate (the compound represented by Formula (I) as discribed below), including Crystal Form I, Crystal Form II, Crystal Form III, Crystal Form IV, Crystal Form V, Crystal Form VI, Crystal Form VII, Crystal Form VIII, Crystal Form IX and Crystal Form X. Further studies unexpectedly revealed that compared to other crystal forms, the Crystal Form II exhibits excellent pharmacokinetic properties, high stability, low hygroscopicity, and good solubility, making it suitable for pharmaceutical uses.

The present invention provides crystal forms of the compound represented by Formula (I) and their uses., wherein the crystal forms can exhibit good stability, good pharmacokinetics, low hygroscopicity and other characteristics, thereby possessing good drugability.

Specifically, the present invention relates to the crystal forms of the compound represented by Formula (I), particularly crystal form II, and pharmaceutical compositions comprising the crystal forms. Furthermore, the invention relates to the use of the crystal forms or pharmaceutical compositions as sGC stimulators, and/or in the manufacture of medicaments for the treatment or prevention of diseases associated with sGC. The crystal forms of the present invention may also exist in the form of solvates, such as hydrates.

In one aspect, the present invention provides a crystal form of the compound represented by formula (I),

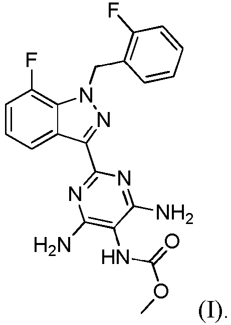

**In** some embodiments, the crystal form of the compound represented by Formula (I) of the present invention is Crystal Form II.

In some embodiments, the Crystal Form II of the present invention is characterized by an X-ray powder diffraction (XRPD) pattern having characteristic peaks at 2θ angles of 7.25°, 11.92°, 12.24°, 15.32°, and 20.73°, wherein the error range of each peak is ± 0.2°.

In some embodiments, the Crystal Form II of the present invention is characterized by an X-ray powder diffraction (XRPD) pattern further having characteristic peaks at at least one of the following 2θ angles: 6.10°, 8.75°, 14.67°, 16.43°, and 29.63°, wherein the error range of each peak is ±0.2°.

In some embodiments, the Crystal Form II of the present invention is characterized by an X-ray powder diffraction (XRPD) pattern having characteristic peaks at 2θ angles of: 6.10°, 7.25°, 8.75°, 11.92°, 12.24°, 14.67°, 15.32°, 16.43°, 20.73°, and 29.63°, wherein the error range of each peak is ±0.2°.

In some embodiments, the Crystal Form II of the present invention is characterized by an X-ray powder diffraction (XRPD) pattern further having characteristic peaks at at least one of the following 2θ angles: 18.37°, 18.84°, 20.06°, 24.00°, 25.79°, 26.03°, 27.44°, 30.98°, 33.41°, 35.13°, 37.26°, 38.77°, 40.98° and 44.53°, wherein the error range of each peak is ±0.2°.

In some embodiments, the Crystal Form II of the present invention is characterized by an X-ray powder diffraction (XRPD) pattern having characteristic peaks at 2θ angles of: 6.10°, 7.25°, 8.75°, 11.92°, 12.24°, 14.67°, 15.32°, 16.43°, 18.37°, 18.84°, 20.06°, 20.73°, 24.00°, 25.79°, 26.03°, 27.44°, 29.63°, 30.98°, 33.41°, 35.13°, 37.26°, 38.77°, 40.98° and 44.53°, wherein the error range of each peak is ±0.2°.

In some embodiments, the Crystal Form II of the present invention is characterized by an X-ray powder diffraction (XRPD) pattern further having characteristic peaks at at least one of the following 2θ angles: 10.50°, 21.03°, 21.86°, 23.49°, 25.00°, 26.49°, 26.93°, 28.68°, 33.41°, 35.56°, 36.35°, 39.62° and 48.16°, wherein the error range of each peak is ±0.2°.

In some embodiments, the Crystal Form II of the present invention is characterized by an X-ray powder diffraction (XRPD) pattern having characteristic peaks at 2θ angles of: 6.10°, 7.25°, 8.75°, 10.50°, 11.92°, 12.24°, 14.67°, 15.32°, 16.43°, 18.37°, 18.84°, 20.06°, 20.73°, 21.03°, 21.86°, 23.49°, 24.00°, 25.00°, 25.79°, 26.03°, 26.49°, 26.93°, 27.44°, 28.68°, 29.63°, 30.98°, 33.41°, 35.13°, 35.56°, 36.35°, 37.26°, 38.77°, 39.62°, 40.98°, 44.53° and 48.16°, wherein the error range of each peak is ±0.2°.

In some embodiments, the Crystal Form II of the present invention is characterized by an X-ray powder diffraction (XRPD) pattern further having characteristic peaks at at least one of the following 2θ angles: 41.69°, 51.26°, 52.76°, 57.61° and 58.92°, wherein the error range of each peak is ±0.2°.

In some embodiments, the Crystal Form II of the present invention is characterized by an X-ray powder diffraction (XRPD) pattern having characteristic peaks at 2θ angles of: 6.10°, 7.25°, 8.75°, 10.50°, 11.92°, 12.24°, 14.67°, 15.32°, 16.43°, 18.37°, 18.84°, 20.06°, 20.73°, 21.03°, 21.86°, 23.49°, 24.00°, 25.00°, 25.79°, 26.03°, 26.49°, 26.93°, 27.44°, 28.68°, 29.63°, 30.98°, 33.41°, 35.13°, 35.56°, 36.35°, 37.26°, 38.77°, 39.62°, 40.98°, 41.69°, 44.53°, 48.16°, 51.26°, 52.76°, 57.61° and 58.92°, wherein the error range of each peak is ±0.2°.

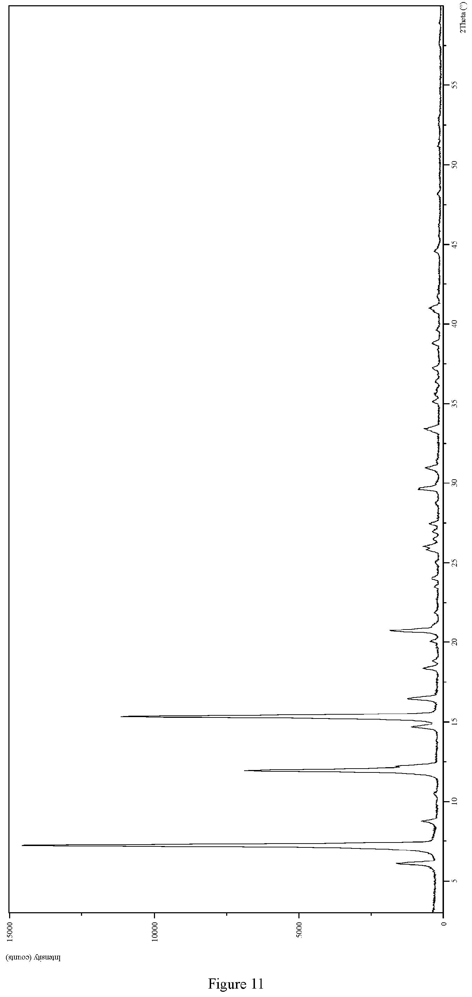

In some embodiments, the Crystal Form II of the present invention is characterized by an X-ray powder diffraction pattern substantially as shown in Figure 11. According to the X-ray powder diffraction analysis, the Crystal Form II exhibits high crystallinity.

In some embodiments, the Crystal Form II of the present invention is characterized by a differential scanning calorimetry (DSC) thermogram comprising an endothermic peak at 269.71°C ± 3°C.

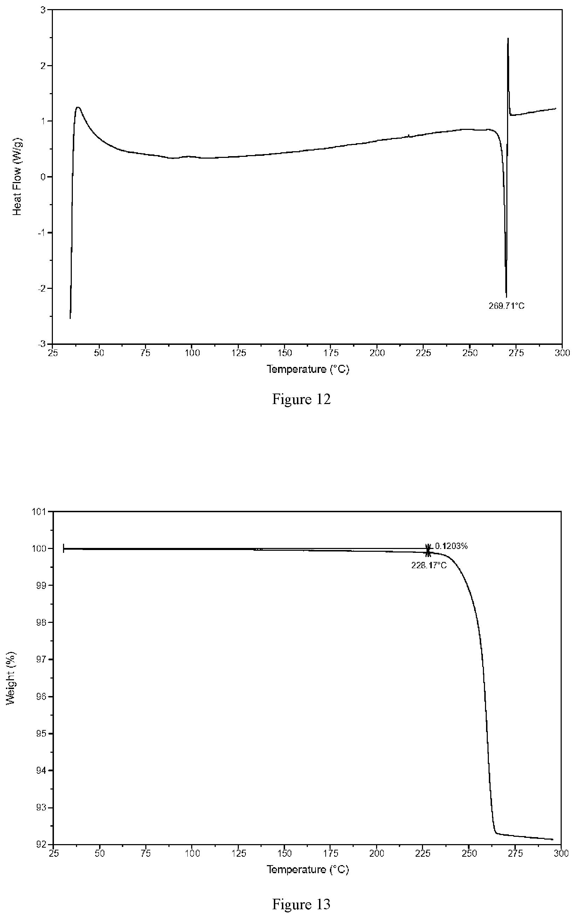

In some embodiments, the Crystal Form II of the present invention is characterized by a differential scanning calorimetry (DSC) thermogram substantially as shown in Figure 12.

In some embodiments, the Crystal Form II of the present invention is characterized in that the Crystal Form II has a weight loss of approximately 0.12% at about 228°C.

In some embodiments, the Crystal Form II of the present invention is characterized by a thermogravimetric analysis (TGA) profile substantially as shown in Figure 13.

In some embodiments, the crystal form of the compound represented by Formula (I) of the present invention is Crystal Form I, Crystal Form III, Crystal Form IV, Crystal Form V, Crystal Form VI, Crystal Form VII, Crystal Form VIII, Crystal Form IX or Crystal Form X.

In some embodiments, the Crystal Form I of the present invention is characterized by an X-ray powder diffraction (XRPD) pattern having characteristic peaks at 2θ angles of: 6.73°, 9.98°, 10.93°, 11.48°, 12.24°, 13.40°, 13.67°, 14.29°, 15.31°, 15.81°, 16.34°, 16.82°, 17.10°, 17.73°, 18.13°, 18.72°, 19.96°, 20.32°, 20.95°, 21.85°, 22.32°, 23.03°, 23.62°, 24.16°, 24.53°, 25.25°, 25.63°, 26.83°, 27.30°, 28.06°, 29.09°, 29.82°, 30.10°, 30.46°, 30.79°, 31.70°, 32.68°, 33.25°, 33.82°, 34.32°, 35.39°, 36.27°, 36.65°, 37.14°, 37.93° and 38.80°, wherein the error range of each peak is ±0.2°.

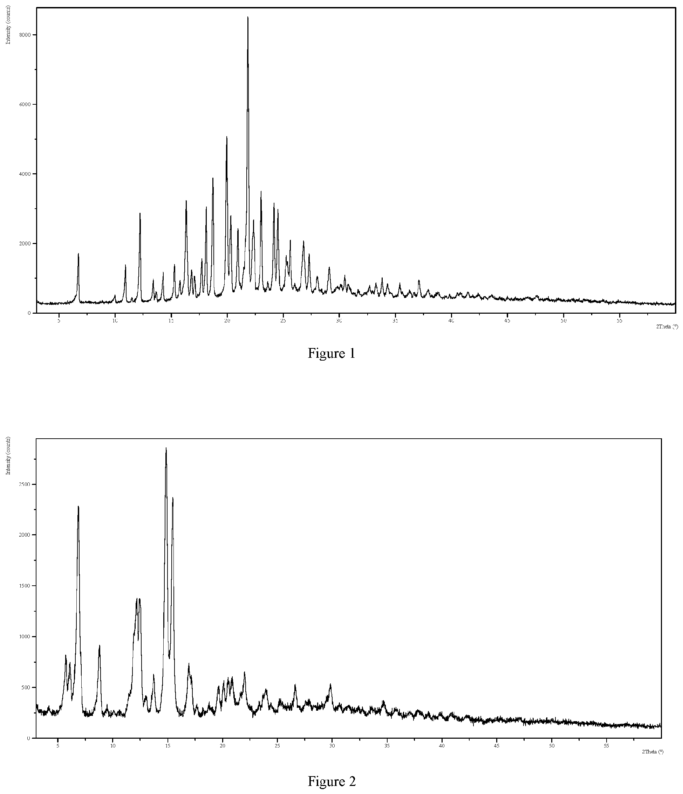

In some embodiments, the Crystal Form I of the present invention is characterized by an X-ray powder diffraction pattern substantially as shown in Figure 1.

In some embodiments, the Crystal Form I of the present invention is characterized by a differential scanning calorimetry (DSC) thermogram comprising endothermic peaks at 159.14°C ± 3°C and 268.85°C ± 3°C.

In some embodiments, the Crystal Form III of the present invention is characterized by an X-ray powder diffraction (XRPD) pattern having characteristic peaks at 2θ angles of: 5.72°, 6.11°, 6.88°, 8.82°, 9.49°, 11.89°, 12.20°, 12.46°, 13.05°, 13.71°, 14.82°, 15.51°, 16.91°, 17.19°, 17.67°, 18.76°, 19.63°, 20.08°, 20.50°, 20.89°, 22.03°, 23.99°, 25.21°, 26.63°, 27.72°, 29.82°, 31.44°, 34.65°, 35.87°, 37.89°, 40.89° and 42.40°, wherein the error range of each peak is ±0.2°.

In some embodiments, the Crystal Form III of the present invention is characterized by an X-ray powder diffraction pattern substantially as shown in Figure 2.

In some embodiments, the Crystal Form III of the present invention is characterized by a differential scanning calorimetry (DSC) thermogram comprising an endothermic peak at 259.12°C ± 3°C.

In some embodiments, the Crystal Form IV of the present invention is characterized by an X-ray powder diffraction (XRPD) pattern having characteristic peaks at 2θ angles of: 6.96°, 7.63°, 11.62°, 12.85°, 13.38°, 13.76°, 13.91°, 14.89°, 16.13°, 16.59°, 17.85°, 19.00°, 19.23°, 19.69°, 20.88°, 21.59°, 21.89°, 23.10°, 23.33°, 24.06°, 24.57°, 25.16°, 26.00°, 26.50°, 27.61°, 28.01°, 28.31°, 29.94°, 30.72°, 31.53°, 32.25°, 32.75°, 33.06°, 34.99°, 36.14°, 36.99°, 38.65°, 40.19°, 42.15°, 45.98°, 47.18°, 47.69°, 48.37°, 49.42°, 50.43°, 51.62°, 54.04° and 54.63°, wherein the error range of each peak is ±0.2°.

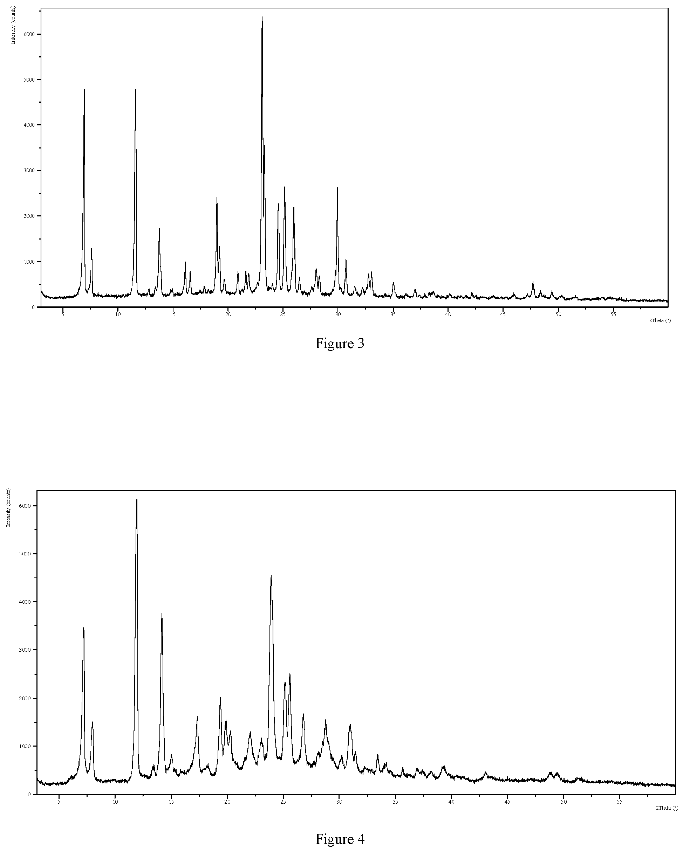

In some embodiments, the Crystal Form IV of the present invention is characterized by an X-ray powder diffraction pattern substantially as shown in Figure 3.

In some embodiments, the Crystal Form IV of the present invention is characterized by a differential scanning calorimetry (DSC) thermogram comprising an endothermic peak at 211.51°C ± 3°C.

In some embodiments, the Crystal Form V of the present invention is characterized by an X-ray powder diffraction (XRPD) pattern having characteristic peaks at 2θ angles of: 7.24°, 8.02°, 11.95°, 13.47°, 14.19°, 15.03°, 17.35°, 18.31°, 19.38°, 19.86°, 20.30°, 22.02°, 22.98°, 23.88°, 24.05°, 25.19°, 25.61°, 26.75°, 28.03°, 28.83°, 30.22°, 30.87°, 31.46°, 32.28°, 33.40°, 34.19°, 35.66°, 36.91°, 37.47°, 38.21°, 39.28°, 43.05°, 48.83°, 49.45° and 51.38°, wherein the error range of each peak is ±0.2°.

In some embodiments, the Crystal Form V of the present invention is characterized by an X-ray powder diffraction pattern substantially as shown in Figure 4.

In some embodiments, the Crystal Form V of the present invention is characterized by a differential scanning calorimetry (DSC) thermogram comprising endothermic peaks at 214.12°C ± 3°C and 268.62°C± 3°C.

In some embodiments, the Crystal Form VI of the present invention is characterized by an X-ray powder diffraction (XRPD) pattern having characteristic peaks at 2θ angles of: 4.25°, 7.97°, 8.29°, 9.83°, 12.71°, 15.72°, 16.95°, 17.39°, 18.39°, 19.54°, 20.19°, 21.20°, 22.84°, 23.66°, 24.11°, 25.23°, 26.18°, 27.13°, 27.79°, 28.17°, 31.40°, 32.50° and 34.83°, wherein the error range of each peak is ±0.2°.

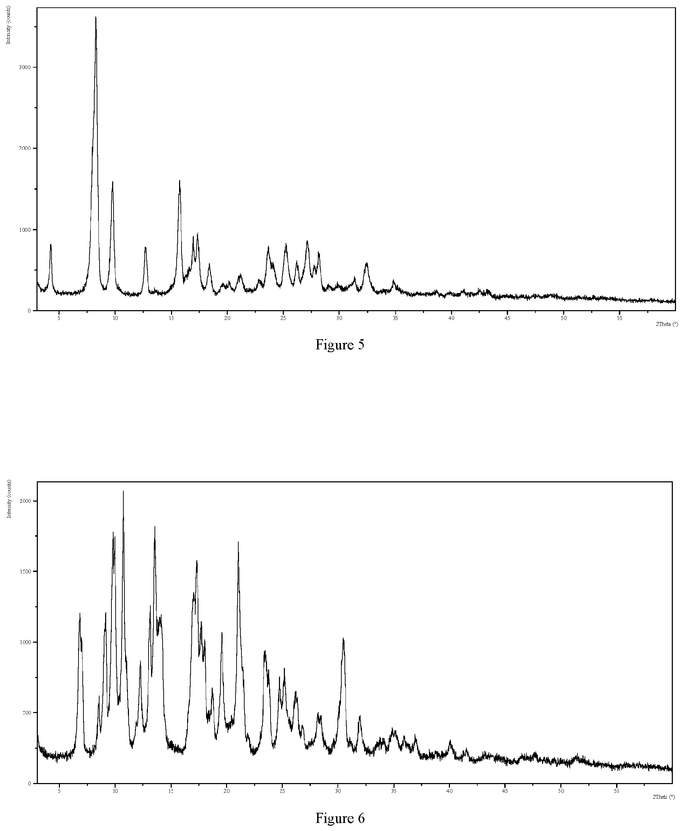

In some embodiments, the Crystal Form VI of the present invention is characterized by an X-ray powder diffraction pattern substantially as shown in Figure 5.

In some embodiments, the Crystal Form VI of the present invention is characterized by a differential scanning calorimetry (DSC) thermogram comprising endothermic peaks at 184.88°C± 3°C and 247.61°C± 3°C.

In some embodiments, the Crystal Form VII of the present invention is characterized by an X-ray powder diffraction (XRPD) pattern having characteristic peaks at 2θ angles of: 6.78°, 7.04°, 8.53°, 9.14°, 9.80°, 9.98°, 10.75°, 12.28°, 13.14°, 13.53°, 14.21°, 16.93°, 17.35°, 17.71°, 18.05°, 18.71°, 19.58°, 21.06°, 21.51°, 23.38°, 23.85°, 24.71°, 25.17°, 26.10°, 26.38°, 26.82°, 28.15°, 28.48°, 30.48°, 31.94°, 33.99°, 34.80°, 35.92°, 36.89°, 40.06°, 41.58°, 47.64° and 51.33°, wherein the error range of each peak is ±0.2°.

In some embodiments, the Crystal Form VII of the present invention is characterized by an X-ray powder diffraction pattern substantially as shown in Figure 6.

In some embodiments, the Crystal Form VII of the present invention is characterized by a differential scanning calorimetry (DSC) thermogram comprising endothermic peaks at 95.38°C± 3°C, 172.09°C+ 3°C and 254.53°C± 3°C.

In some embodiments, the Crystal Form VIII of the present invention is characterized by an X-ray powder diffraction (XRPD) pattern having characteristic peaks at 2θ angles of: 5.64°, 7.47°, 9.64°, 10.03°, 12.36°, 14.65°, 15.50°, 16.66°, 17.01°, 17.68°, 18.27°, 19.16°, 20.13°, 22.05°, 22.64°, 23.84°, 24.15°, 25.41°, 26.50°, 27.27°, 27.96°, 29.45°, 29.98°, 31.21°, 32.19°, 33.62°, 34.31°, 35.20°, 36.76°, 37.45°, 38.74°, 39.33°, 39.95°, 42.01°, 44.55°, 46.94°, 48.39° and 51.09°, wherein the error range of each peak is ±0.2°.

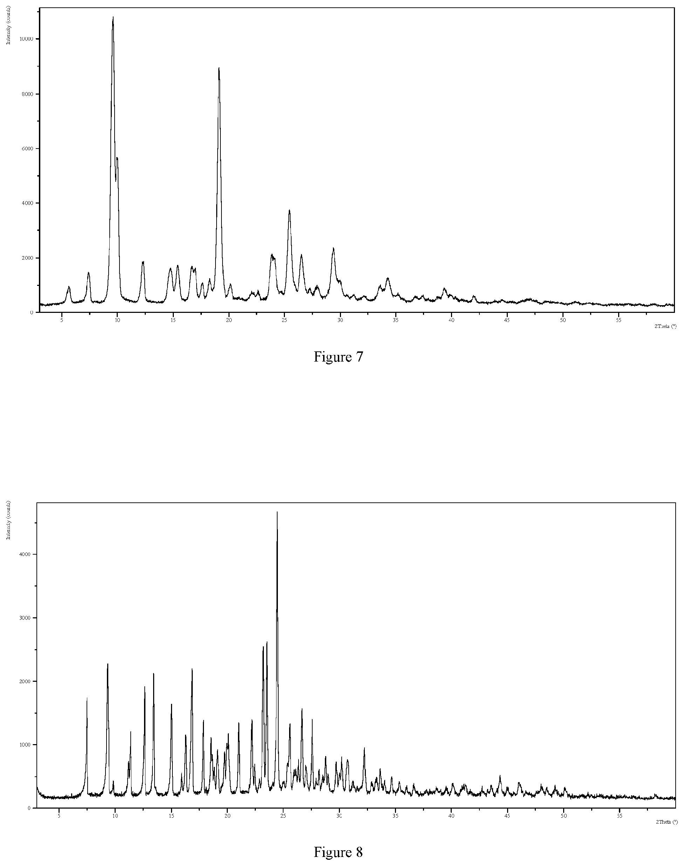

In some embodiments, the Crystal Form VIII of the present invention is characterized by an X-ray powder diffraction pattern substantially as shown in Figure 7.

In some embodiments, the Crystal Form VIII of the present invention is characterized by a differential scanning calorimetry (DSC) thermogram comprising endothermic peaks at 140.23°C± 3°C, 171.86°C± 3°C and 259.47°C± 3°C.

In some embodiments, the Crystal Form IX of the present invention is characterized by an X-ray powder diffraction (XRPD) pattern having characteristic peaks at 2θ angles of: 9.32°, 16.86°, 23.21°, 23.53° and 24.46°, wherein the error range of each peak is ±0.2°.

In some embodiments, the Crystal Form IX of the present invention is characterized by an X-ray powder diffraction (XRPD) pattern having characteristic peaks at 2θ angles of: 7.49°, 9.32°, 12.63°, 13.45°, 15.04°, 16.86°, 23.21°, 23.53°, 24.46° and 26.65°, wherein the error range of each peak is ±0.2°.

In some embodiments, the Crystal Form IX of the present invention is characterized by an X-ray powder diffraction (XRPD) pattern having characteristic peaks at 2θ angles of: 7.49°, 9.32°, 9.84°, 11.18°, 11.39°, 12.63°, 13.45°, 15.04°, 15.94°, 16.30°, 16.86°, 17.87°, 18.54°, 18.68°, 18.84°, 19.09°, 19.74°, 19.97°, 20.09°, 21.02°, 22.19°, 22.44°, 22.88°, 23.21°, 23.53°, 24.46°, 25.35°, 25.58°, 25.93°, 26.34°, 26.65°, 26.98°, 27.56°, 27.93°, 28.15°, 28.76°, 29.00°, 29.71°, 30.19°, 30.79°, 31.20°, 32.25°, 32.86°, 33.33°, 33.63°, 34.04°, 34.64°, 35.35°, 35.98°, 36.63°, 37.73°, 38.67°, 39.54°, 40.11°, 41.28°, 42.78°, 43.54°, 44.32°, 44.95°, 46.00°, 48.05°, 48.48°, 49.24°, 50.09° and 58.19°, wherein the error range of each peak is ±0.2°.

In some embodiments, the Crystal Form IX of the present invention is characterized by an X-ray powder diffraction pattern substantially as shown in Figure 8.

In some embodiments, the Crystal Form IX of the present invention is characterized by a differential scanning calorimetry (DSC) thermogram comprising endothermic peaks at 161.94°C± 3°C and 268.77°C± 3°C.

In some embodiments, the Crystal Form X of the present invention is characterized by an X-ray powder diffraction (XRPD) pattern having characteristic peaks at 2θ angles of: 5.63°, 7.04°, 9.98°, 10.37°, 11.48°, 13.12°, 15.17°, 16.00°, 17.19°, 17.73°, 18.76°, 19.91°, 20.64°, 21.65°, 22.88°, 25.04°, 25.55°, 26.35°, 27.42°, 28.67°, 30.16°, 32.02°, 32.54°, 33.87°, 35.17°, 36.15°, 37.90°, 40.31°, 41.90°, 42.65°, 43.83°, 45.72°, 46.56°, 51.62°, 52.35° and 53.12°, wherein the error range of each peak is ±0.2°.

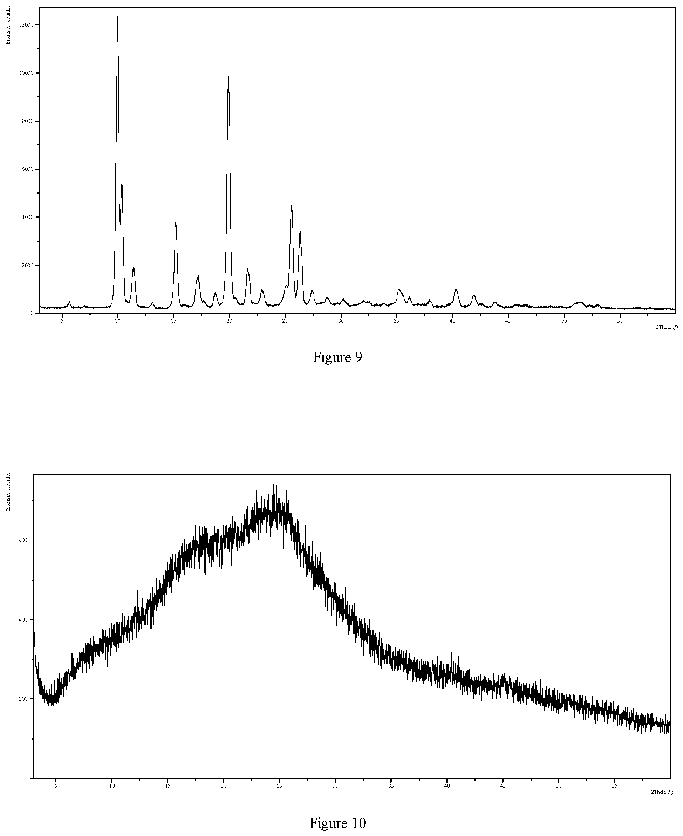

In some embodiments, the Crystal Form X of the present invention is characterized by an X-ray powder diffraction pattern substantially as shown in Figure 9.

In some embodiments, the Crystal Form X of the present invention is characterized by a differential scanning calorimetry (DSC) thermogram comprising endothermic peaks at 175.16°C± 3°C and 252.36°C± 3°C.

On the other hand, the present invention further provides an amorphous form of the compound represented by Formula (I), which has an X-ray powder diffraction (XRPD) pattern substantially as shown in Figure 10.

In one aspect, the present invention relates to a preparation method of the Crystal Form II of the compound represented by Formula (I), comprising:
adding Crystal Form I or Crystal Form IX of the compound represented by Formula (I) to a solvent, slurrying, and filtering to obtain the target crystal form.

In some embodiments, the Crystal Form I or Crystal Form IX used in the preparation method of the Crystal Form II is as described in the present invention. For example, the Crystal Form I has an XRPD pattern substantially as shown in Figure 1, the Crystal Form IX has an XRPD pattern substantially as shown in Figure 8.

In some embodiments, in the preparation method of the Crystal Form II of the compound represented by Formula (I) of the present invention, the solvent may be acetone. For example, the Crystal Form I or Form IX of the compound represented by Formula (I) is added to acetone.

In some embodiments, in the preparation method of the Crystal Form II of the compound represented by Formula (I) of the present invention, the slurrying may be performed at a certain temperature, for example, at approximately 60°C. In other embodiments, the slurrying is maintained for a period of time, which may be adjusted as needed, including but not limited to 8 hours.

In some embodiments, in the preparation method of the Crystal Form II of the compound represented by Formula (I) of the present invention, the filtration may be suction filtration. In further embodiments, the filter cake obtained from filtration is washed with a corresponding solvent.

In some embodiments, the filtered solid (i.e., filter cake) is dried to obtain the target crystal form. In some embodiments, the drying may be vacuum drying; in other embodiments, it may be high-temperature vacuum drying, optionally, the high temperature refers to heating to a certain temperature, such as approximately 45°C to 60°C.

On the other hand, the present invention relates to a second preparation method of the Crystal Form II of the compound represented by Formula (I), comprising: dissolving the compound represented by Formula (I) in Solvent 1 or a mixed solvent of Solvent 1 and Solvent 2, then adding Solvent 2, cooling, filtering to obtain the target crystal form.

In some embodiments, in the second preparation method of the Crystal Form II of the compound represented by Formula (I), the solvent 1 may be N,N-dimethylformamide , dimethyl sulfoxide, or the like. In other embodiments, the solvent 2 may be ethyl acetate, sec-butanol, n-butanol, or the like.

In some embodiments, in the second preparation method of the Crystal Form II of the compound represented by Formula (I), the compound represented by Formula (I) is added to Solvent 1 or the mixed solvent of Solvent 1 and Solvent 2 and heated to dissolve. In other embodiments, the heating is performed at about 70°C to 95°C, such as about about 75°C, about 77°C, about 80°C, about 85°C, or about 90°C.

In some embodiments, in the second preparation method of the Crystal Form II of the compound represented by Formula (I), after dissolution of the compound represented by Formula (I), the solution is maintained at a constant temperature for a period of time. In some embodiments, the term "maintaining at a constant temperature" refers to keeping the temperature of the solution system steady. In some embodiments, maintaining at a constant temperature for a period of time may refer to maintaining it for about 10 minutes to 30 minutes, for example, about 10 minutes to 20 minutes.

The maintenance time is not strictly limited and can be adjusted based on factors such as the feedstock quantity.

In some embodiments, in the second preparation method of the Crystal Form II of the compound represented by Formula (I), after adding Solvent 2, the resulting solution system is maintained at temperature for a period of time.

In some embodiments, in the second preparation method of the Crystal Form II of the compound represented by Formula (I), the cooling may be natural cooling, for example, naturally cooling to room temperature; or cooling to room temperature at a defined rate, for example, at a rate of 5°C per 20 minutes. In some embodiments, in the second preparation method of the Crystal Form II of the compound represented by Formula (I), after cooling to room temperature, the solution system may continued stirring, for example, for an additional period.

In some embodiments, in the second preparation method of the Crystal Form II of the compound represented by Formula (I), the filtration may be suction filtration.

In some embodiments, after filtration, the filter cake is washed with Solvent 2.

In some embodiments, the filtered solid (i.e., filter cake) is dried to obtain the target crystal form. In some embodiments, the drying may be vacuum drying; in other embodiments, it may be high-temperature vacuum drying, optionally, the high temperature refers to heating to a certain temperature, such as approximately 45°C to 60°C.

The preparation method of the Crystal Form II of the compound represented by Formula (I) described in the present invention has simple operation, high yield and high product purity Particularly, the method for preparing Crystal Form II by using Crystal Form I or Crystal Form IX disclosed herein is simpler to operate and can afford the target crystal form with higher yield and exceptional purity.

In one aspect, the invention relates to a pharmaceutical composition comprising any one of the crystal forms or amorphous form of the compound represented by Formula (I) provided herein, and one or more pharmaceutically acceptable carriers, excipients, diluents, adjuvants, or combinations thereof.

In some embodiments, the crystal form is Crystal Form **II,** which has the meanings as defined in the invention, or is prepared by any one of the preparation methods of Crystal Form II described herein.

In some embodiments, the composition may further include one or more additional active ingredients.

In another aspect, the invention involves the use of any crystal form (such as Crystal Form II), or the amorphous form, or the pharmaceutical composition described herein in the manufacture of a medicament for treating and/or preventing heart failure, angina pectoris, hypertension, pulmonary hypertension, ischemia, vascular disorders, kidney disease, thromboembolic disease, male sexual dysfunction, systemic sclerosis, sickle cell anemia, achalasia, fibrotic disease, and/or arteriosclerosis.

On the other hand, the present invention relates to the use of any one of the crystal forms (such as Crystal Form II), or the amorphous form, or the pharmaceutical composition of the present invention in the manufacture of a medicament, wherein the medicament is used as a soluble guanylate cyclase stimulator.

On one hand, the present invention relates to a method for treating, alleviating and/or preventing a disease in patients, wherein the disease is heart failure, angina pectoris, hypertension, pulmonary hypertension, ischemia, vascular disease, kidney diseases, thromboembolic disease, male sexual dysfunction, systemic sclerosis, sickle cell anemia, achalasia, fibrotic diseases and/or arteriosclerosis; the method comprises administering to the patient an effective pharmaceutical dose of any one of the crystal forms (e.g. Crystal Form II), the amorphous form or the pharmaceutical composition described in the present invention.

In another aspect, the present invention relates to a method for stimulating soluble guanylate cyclase (sGC), comprising administering an effective dose of any crystal form (e.g., Crystal Form II), the amorphous form, or the pharmaceutical composition described herein to a subject in need thereof.

In one aspect, the invention relates to any one of the crystal forms (e.g., Crystal Form II), or the amorphous form, or the pharmaceutical composition decribed herein for use in treating and/or preventing heart failure, angina pectoris, hypertension, pulmonary hypertension, ischemia, vascular disorders, kidney disease, thromboembolic disease, male sexual dysfunction, systemic sclerosis, sickle cell anemia, achalasia, fibrotic disease, and/or arteriosclerosis.

On the other hand, the present invention relates to any one of the crystal forms (such as Crystal Form II), the amorphous form, or the pharmaceutical composition provided herein for use as a soluble guanylate cyclase stimulator.

The present invention provides activity test experiments (e.g., a pharmacokinetic experiment), solubility tests, stability tests, and hygroscopicity experiments for the crystal forms described herein.

Through research, it has been found that some crystal forms of the compound represented by formula (I) of the present invention are unstable, including but not limited to undergoing crystal form transformation, i.e., polymorphic transition. For example, Crystal Form V undergoes polymorphic transition under high temperature conditions; similarly, Crystal Forms I, IV, V and IX transform into Crystal form II in solvents such as ethanol. In contrast, Crystal Form II has a stable crystal structure and is less prone to polymorphic transition, indicating that Crystal Form II of the present invention is more stable. Moreover, experiments have proven that Crystal Form II of the present invention exhibits excellent stability, effectively preventing changes in crystal structure, bioavailability, and pharmacodynamic properties during drug storage and development. The crystal form is also less susceptible to deliquescence under high humidity conditions, facilitating long-term drug storage; furthermore, the Crystal Form II demonstrates good solubility, which is beneficial for enhancing pharmacodynamic properties and reducing drug loading. At the same time, Crystal Form II possesses favorable biological activity.

In summary, the Crystal Form II described in the present invention exhibits good biological activity, excellent solubility and high stability, making it suitable for pharmaceutical uses.

### DEFINITIONS AND GENERAL TERMINOLOGY

Unless otherwise indicated, all technical and scientific terms used in the present invention have the same meaning as commonly understood by one of ordinary skill in the art to which this invention pertains. All patents and publications referred to herein are incorporated by reference in their entirety. Although any methods and materials similar or identical to those described herein may be used in the practice or testing of the invention, but the methods, apparatus and materials described in the invention are preferred.

"Crystal form" or "crystalline form" refers to a solid having a highly regular chemical structure, including, but not limited to, mono- or multi-component crystals, and/or polymorphic compounds of compounds, solvates, hydrates, clathrates, eutectic, salt, salt solvent, salt hydrate. The crystalline form of the material can be obtained by a number of methods known in the field. Such methods include, but are not limited to, melt crystallization, melt cooling, solvent crystallization, crystallization in defined space, for example, in nanopores or capillaries, on a surface or template, for example, on a polymer, in the presence of additives such as co-crystallization counterions, removing solvent, dehydration, rapid evaporation, rapid cooling, slow cooling, vapor diffusion, sublimation, reaction crystallization, anti-solvent addition, grinding and solvent drop milling.

"Solvent" refers to a substance (typically a liquid) that is capable of completely or partially dissolving another substance (typically a solid). Solvents for use in the practice of this invention include, but are not limited to, water, acetic acid, acetone, acetonitrile, benzene, chloroform, carbon tetrachloride, dichloromethane, dimethylsulfoxide, 1,4-dioxane, , Ethyl acetate, butanol, t-butanol, N, N-dimethylacetamide, N, N-dimethylformamide, formamide, formic acid, heptane, hexane, isopropanol, Methyl ethyl ketone, mesitylene, nitromethane, polyethylene glycol, propanol, pyridine, tetrahydrofuran, toluene, xylene, mixtures thereof, and the like.

"Solvate" refers to a compound that on a surface, in a lattice or having a solvent on a surface or in a lattice. The solvent can be water, acetic acid, acetone, acetonitrile, benzene, chloroform, carbon tetrachloride, dichloromethane, dimethylsulfoxide, 1,4-dioxane, ethanol, ethyl acetate, butanol, t-butanol, N, N-dimethylacetamide, N, N-dimethylformamide, formamide, formic acid, heptane, hexane, isopropanol, methanol, methyl ethyl ketone, methyl pyrrolidone, mesitylene, nitromethane, polyethylene glycol, propanol, pyridine, tetrahydrofuran, toluene, xylene, mixtures thereof, and the like. A specific example of the solvate is a hydrate in which the solvent on the surface, in the lattice or on the surface and in the lattice is water. On the surface, in the lattice or on the the surface and in the lattic of the substance, the hydrate may or may not have any solvent other than water.

Crystal form can be identified by a variety of technical means, such as X-ray powder diffraction (XRPD), infrared absorption spectroscopy (IR), melting point method, differential scanning calorimetry (DSC), thermogravimetric analysis (TGA), Nuclear magnetic resonance, Raman spectroscopy, X-ray single crystal diffraction, dissolution calorimetry, scanning electron microscopy (SEM), quantitative analysis, solubility and dissolution rate, and the like.

X-ray powder diffraction (XRPD) can detect changes in crystal form, crystallinity, crystal state and other information, is a common means for identifying crystal form. The peak position of the XRPD pattern primarily depends on the structure of the crystal form and is relatively insensitive to the experimental details, and its relative peak height depends on many factors associated with sample preparation and instrument geometry. In some embodiments, the crystal form of the present invention is characterized by an XRPD map having certain peak positions, which is substantially as shown in the XRPD diagram provided in the drawings of the present invention At the same time, the 2θ of the XRPD pattern can be measured with an experimental error. The measurement of 2θ of the XRPD pattern may be slightly different between the different instruments and the different samples. Therefore, the value of 2θ can not be regarded as absolute. According to the condition of the instrument used in this test, the diffraction peak has an error tolerance of ± 0.2°^{.}

Differential Scanning Calorimetry (DSC) is a technique of measuring the energy difference between a sample and an inert reference (commonly used α-Al₂O₃) with temperature by continuously heating or cooling under program control. The high endothermic peak of the DSC thermogram depends on many factors associated with sample preparation and instrument geometry, while the peak position is relatively insensitive to experimental details. Thus, in some embodiments, the crystal form of the present invention is characterized by an DSC map having certain peak positions, which is substantially as shown in the DSC diagram provided in the drawings of the present invention. At the same time, the DSC pattern can be measured with an experimental error. The peak position and peak value of DSC pattern may be slightly different between the different instruments and the different samples. Therefore, the peak position or the peak value of the DSC endothermic peak can not be regarded as absolute. Based on the conditions of the instrument used in this test, the endothermic peaks have an error tolerance of ±3°C.

Thermogravimetric analysis (TGA) is a technique that measures the mass change of a substance as a function of temperature under programmed control. It is suitable for examining the loss of solvent in crystals or processes such as sublimation and decomposition, and can be used to infer the presence of crystalline water or solvent in the crystal. The quality variety of the TGA curve shown depend on a number of factors, contains the sample preparation and the instrument. The quality variety of the TGA test may be slightly different between the different instruments and between the different samples. According to the condition of the instrument used in this test, there is a ± 0.1% error tolerance for the mass change.

In the context of the present invention, the 2θ values in the X-ray powder diffraction pattern are in degrees (°).

The term "substantially as shown in the figure" refers to at least 50%, or at least 60%, or at least 70%, or at least 80%, or at least 80% of At least 90%, or at least 95%, or at least 99% of the peaks are shown in the X-ray powder diffraction pattern or DSC pattern or Raman spectra pattern or infrared spectra pattern.

The "peak" refers to a feature that a person skilled in the art can recognize without belonging to background noise when referring to a spectrum or/and data that appears in the figure.

The present invention relates to a new crystal form of the compound represented by Formula (I), for example, Crystal Form II, which exists in a substantially pure crystal form.

"Substantially pure" means that a crystalline form is substantially free of another or more crystalline forms, that means the purity of the crystalline form is at least 80%, or at least 85%, or at least 90%, or at least 93%,or at least 95%, or at least 99%, or at least 99%, or at least 99.5%, or at least 99.6%, or at least 99.7%, or at least 99.8%, or at least 99.9%, or crystal form containing other crystal form. The percentage of the other crystals in the total volume or total weight of the crystal form is less than 20%, or less than 10%, or less than 5%, or less than 3%, or less than 1%, or less than 0.5%, or less than 0.1%, or less than 0.01%.

"Substantially free" means that the percentage of one or more other crystalline forms in the total volume or total weight of the crystalline form is less than 20%, or less than 10%, or less than 5%, or less than 4% , or less than 3%, or less than 2%, or less than 1%, or less than 0.5%, or less than 0.1%, or less than 0.01%.

In XRPD patterns, "relative intensity" (or "relative peak height") refers to the ratio of the intensity of other peaks to that of the strongest peak when the intensity of the strongest peak in the X-ray powder diffraction pattern (XRPD) is set as 100%.

In the context of the present invention, when used or whether or not used the word, such as "about", it means that within a given value or range of 10% or less, appropriately within 5%, especially within 1% . Or, for those of ordinary skill in the art, the term"about" means within an acceptable standard error range of the mean value. When a number with an N value is made public, any number within N +/- 1%, N +/- 2%, N +/- 3%, N +/- 5%, N +/- 7%, N +/- 8%, or N +/- 10% will be opened clearly, wherein "+/-" means plus or minus.

In the present invention, "room temperature" refers to the temperature from approximately 10 °C to approximately 40 °C. In some embodiments, "room temperature" refers to a temperature ranging from approximately 20°C to approximately 30°C; in other embodiments, "room temperature" refers to 20°C, 22.5°C, 25°C, 27.5°C, and the like.

As used herein, the"treat", "treating" or "treatment" of any disease or disorder refers in some embodiments, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (e.g., stabilization of a discernible symptom), physiologically, (e.g., stabilization of a physical parameter), or both. In yet another embodiment, "treat", "treating" or "treatment" refers to preventing or delaying the onset or development or progression of the disease or disorder.

### Pharmaceutical compositions, formulations, administration and uses

The pharmaceutical composition of the present invention is characterized by comprising any crystal form or amorphous form of the compound represented by Formula (I), together with a pharmaceutically acceptable carrier, adjuvant, or excipient.

In the pharmaceutical composition of the present invention, the amount of the crystal form of the compound is therapeutically effective and detectably sufficient to treat or alleviate a patient's sGC-related disorder.

As described above, the pharmaceutical compositions disclosed herein further comprise a pharmaceutically acceptable carrier, an adjuvant, or a vehicle, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. As described in the following: In Remington: According to The Science and Practice of Pharmacy, 21st edition (2005, ed. D.B. Troy, Lippincott Williams & Wilkins, Philadelphia) and Encyclopedia of Pharmaceutical Technology (eds. J. Swarbrick and J.C. Boylan, 1988-1999, Marcel Dekker, New York), various carriers can be used in the formulation of pharmaceutically acceptable compositions, along with their well-established preparation methods. Except insofar as any conventional carrier medium incompatible with the crystal forms of compounds disclosed herein, such as by producing any undesirable biological effect or otherwise interacting in a deleterious manner with any other components of the pharmaceutically acceptable composition, its use is contemplated to be within the scope of this invention.

Some non-limiting examples of materials which can serve as pharmaceutically acceptable carriers include ion exchangers; aluminium; aluminum stearate; lecithin; serum proteins such as human serum albumin; buffer substances such as phosphates; glycine; sorbic acid; potassium sorbate; partial glyceride mixtures of saturated vegetable fatty acids; water; salts or electrolytes such as protamine sulfate, disodium hydrogen phosphate, potassium hydrogen phosphate, sodium chloride and zinc salts; colloidal silica; magnesium trisilicate; polyvinyl pyrrolidone; polyacrylates; waxes; polyethylene-polyoxypropylene-block polymers; wool fat; sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; and phosphate buffer solutions, as well as other non-toxic compatible lubricants such as sodium lauryl sulfate and magnesium stearate, as well as coloring agents, releasing agents, coating agents, sweetening, flavoring , perfuming agents, preservatives and antioxidants.

The pharmaceutical composition of the present invention may be formulated as capsules, tablets, pills, powders, granules, or aqueous suspensions/solutions. It can be administered via the following routes: oral, injection, spray inhalation, topical, rectal, nasal, buccal, vaginal, or through an implantable drug reservoir.

For oral administration, the composition may be delivered in forms such as tablets, pills, capsules, dispersible powders, granules, suspensions, syrups, or elixirs. For topical administration, it may be formulated as ointments, gels, medicated patches, etc.

The crystalline or amorphous form of the present invention is preferably formulated into dosage units according to pharmaceutical formulations to minimize dosing variability and enhance dose uniformity. The term "dosage unit type" herein refers to a physically dispersed unit in which a patient obtains the appropriate treatment. It should be understood that the total daily dosage of the crystalline or amorphous form of the compound represented by Formula (I) or the pharmaceutical composition of the present invention shall be determined by the attending physician within the bounds of sound medical judgment. The specific effective dose level for any particular patient or organism will depend on a number of factors including the severity of the disease and condition being treated, the activity of the crystalline or amorphous form of particular compound, the particular composition used, the age, weight, health, sex and eating habits of the patient, time of administration, route of administration and excretion rate of the crystalline or amorphous form of particular compound used, duration of treatment, drug use in combination or in combination with a crystalline or amorphous form of specific compound, and other well known factors in the field of pharmacy.

The effective dosage of the active ingredient may vary depending on the specific crystalline or amorphous form employed, the mode of administration, and the severity of the condition being treated. Typically, satisfactory therapeutic outcomes are achieved when administering the crystalline or amorphous form of the compound at a daily dose ranging from approximately 0.25 to 1000 mg/kg of body weight. The preferred regimen comprises divided doses administered 2-4 times daily, or sustained-release formulation administration. This dosing regimen may be adjusted to achieve optimal therapeutic response. Furthermore, based on individual therapeutic requirements, the total daily dose may be administered as multiple divided doses, or the dose may be proportionally reduced.

The crystalline or amorphous forms of the compound may be administered as monotherapy, or in combination with other active compounds when clinically indicated. The invention further provides combination therapies comprising at least one crystalline or amorphous form of the compound according to the invention, and one or more additional active agents, particularly for the treatment and/or prevention of the diseases as described herein.

The crystalline or amorphous forms of the compound of the present invention serve as soluble guanylate cyclase (sGC) stimulators, demonstrating comparable or superior therapeutic profiles versus prior art, including equivalent or improved: in vivo performance, pharmacokinetic and pharmacodynamic properties, dose-activity relationships and/or safety profiles. These forms are indicated for the treatment and/or prevention of diseases in humans and animals.

The crystalline/amorphous forms or pharmaceutical compositions of the invention are particularly suitable for treating and/or preventing cardiovascular disorders, pulmonary conditions, thromboembolic diseases and fibrotic pathologies.

Specifically, the crystalline form or amorphous form of the compound involved in the present invention, or the pharmaceutical composition thereof, can be used for treating or preventing the following diseases: cardiovascular disease, such as hypertension, acute and chronic heart failure, coronary heart disease, stable and unstable angina pectoris, peripheral vascular and cardiovascular disease, arrhythmias, atrial and ventricular arrhythmias, and impaired conduction (e.g., **I-III** degree atrioventricular block (AV block I-III)), supraventricular tachyarrhythmias, atrial fibrillation, atrial flutter, ventricular fibrillation, ventricular flutter, ventricular tachyarrhythmias, torsades de pointes, atrial and ventricular premature beats, AV junctional premature beats, sick sinus syndrome, syncope, AV nodal reentrant tachycardia, Wolff-Parkinson-White syndrome; acute coronary syndrome (ACS), autoimmune heart diseases (pericarditis, endocarditis, Valvolitis, aortitis, cardiomyopathy), shock such as cardiogenic shock, septic shock and anaphylactic shock, aneurysm, boxer's cardiomyopathy (premature ventricular contraction (PVC)); thromboembolic diseases and ischemic conditions, such as myocardial ischemia, myocardial infarction, stroke, cardiac hypertrophy, transient ischemic attacks, preeclampsia, inflammatory cardiovascular diseases, coronary and peripheral arterial spasms, edema formation (e.g., pulmonary edema, cerebral edema, renal edema, or edema caused by heart failure), peripheral circulation disorders, reperfusion injury, arterial and venous thrombosis, microalbuminuria, myocardial dysfunction, endothelial dysfunction; prevention of restenosis after thrombolytic therapy, percutaneous transluminal angioplasty (PTA), percutaneous transluminal coronary angioplasty (PTCA), heart transplantation, and shunt surgery, as well as microvascular and macrovascular injuries (vasculitis), increased fibrinogen and low-density lipoprotein (LDL) levels, and elevated plasminogen activator inhibitor-1 (PAI-1) concentrations; erectile dysfunction and female sexual dysfunction.

For the purposes of the present invention, the term "heart failure" also includes more specific or related disease types, such as acute decompensated heart failure, right heart failure, left heart failure, global failure, ischemic cardiomyopathy, dilated cardiomyopathy, hypertrophic cardiomyopathy, idiopathic cardiomyopathy, congenital heart defects, valvular heart disease, heart failure associated with valvular heart disease, mitral stenosis, mitral regurgitation, aortic stenosis, aortic regurgitation, tricuspid stenosis, tricuspid regurgitation, pulmonary valve stenosis, pulmonary valve regurgitation, combined valvular defects, myocardial inflammation (myocarditis), chronic myocarditis, acute myocarditis, viral myocarditis, diabetic heart failure, alcoholic cardiomyopathy, cardiac storage diseases, and diastolic and systolic heart failure.

Furthermore, the crystalline or amorphous forms of the compound according to the present invention, or the pharmaceutical compositions thereof, are also suitable for treating and/or preventing: asthma; pulmonary arterial hypertension (PAH) and other forms of pulmonary hypertension (PH) (including PH associated with left heart disease, HIV, sickle cell anemia, chronic thromboembolism (CTEPH), sarcoidosis, COPD, or pulmonary fibrosis); chronic obstructive pulmonary disease (COPD); acute respiratory distress syndrome (ARDS); acute lung injury (ALI); alpha-1 antitrypsin deficiency (AATD); pulmonary fibrosis; emphysema (e.g., smoking-induced emphysema); and cystic fibrosis (CF).

### Description of the drawings

Figure 1 shows the X-ray powder diffraction (XRPD) pattern of Crystal Form I of the compound represented by Formula (I).
Figure 2 shows the X-ray powder diffraction (XRPD) pattern of Crystal Form III of the compound represented by Formula (I).
Figure 3 shows the X-ray powder diffraction (XRPD) pattern of Crystal Form IV of the compound represented by Formula (I).
Figure 4 shows the X-ray powder diffraction (XRPD) pattern of Crystal Form V of the compound represented by Formula (I).
Figure 5 shows the X-ray powder diffraction (XRPD) pattern of Crystal Form VI of the compound represented by Formula (I).
Figure 6 shows the X-ray powder diffraction (XRPD) pattern of Crystal Form VII of the compound represented by Formula (I).
Figure 7 shows the X-ray powder diffraction (XRPD) pattern of Crystal Form VIII of the compound represented by Formula (I).
Figure 8 shows the X-ray powder diffraction (XRPD) pattern of Crystal Form IX of the compound represented by Formula (I).
Figure 9 shows the X-ray powder diffraction (XRPD) pattern of Crystal Form X of the compound represented by Formula (I).
Figure 10 shows the X-ray powder diffraction (XRPD) pattern of amorphous Form of the compound represented by Formula (I).
Figure 11 shows the X-ray powder diffraction (XRPD) pattern of Crystal Form II of the compound represented by Formula (I).
Figure 12 presents the differential scanning calorimetry (DSC) thermogram of Crystal Form II of the compound represented by Formula (I).
Figure 13 displays the thermogravimetric analysis (TGA) profile of Crystal Form II of the compound represented by Formula (I).
Figure 14 illustrates the dynamic vapor sorption (DVS) isotherm of Crystal Form II of the compound represented by Formula (I).

### EXAMPLES

The invention will now be further described by way of example without limiting the invention to the scope of the invention.

The X-ray powder diffraction analysis method used in the present invention was an Empyrean diffractometer, and an X-ray powder diffraction pattern was obtained using Cu-Kα radiation (45 KV, 40 mA). The powdery sample was prepared as a thin layer on a monocrystalline silicon sample rack and placed on a rotating sample stage, analyzed at a rate of 0.0167 steps in the range of 3 °-60 °. Use Data Collector software to collect data, HighScore Plus software to process data, Data Viewer software to read data.

The differential scanning calorimetry (DSC) analysis method used in the present invention is a differential scanning calorimeter using a TA Q2000 module with a thermal analysis controller. Data were collected and analyzed using TA Instruments Thermal Solutions software. Approximately 1-5 mg of the sample was accurately weighed into a specially crafted aluminum crucible with a lid and analyzed from room temperature to about 300 ° C using a linear heating device at 10 ° C/min. During use, the DSC chamber was purged with dry nitrogen.

The thermogravimetric analysis (TGA) method used in the present invention was performed using a TA Q500 module equipped with a thermal analysis controller. Data were collected and analyzed using TA Instruments Thermal Solutions software. Approximately 10 mg of sample was accurately weighed into a platinum sample pan and analyzed using a linear heating rate of 10°C/min from room temperature to approximately 300°C. During operation, the TGA furnace chamber was purged with dry nitrogen.

The solubility of the present invention was determined using an Agilent 1200 High Performance Liquid Chromatograph DAD/VWD detector with an Agilent XDB-C18 model (4.6 x 50 mm, 5 µm). Detection wavelength of 266 nm, flow rate of 1.0 mL/min, the column temperature of 35 °C, mobile phase A: acetonitrile - 0.01 M ammonium acetate = 10:90 (V: V) analysis methods: acetonitrile - mobile phase A= 70:30 (V: V), running time: 10 minutes.

The hygroscopicity of the present invention was determined using a DVS INT-Std dynamic vapor and gas sorption analyzer from Surface Measurement Systems Ltd. (UK) with the following parameters: humidity test range: 0%-95%, gas flow: 200 mL/min, temperature: 25°C, test points: one measurement point every 5% humidity increase.

### EXAMPLES

The present invention provides preparation examples of crystalline or amorphous forms of the compound of Formula (I). Skilled in the art can learn from this article to properly improve the process parameters to implement the preparation method. Of particular note is that all similar substitutions and modifications to the skilled person is obvious, and they are deemed to be included in the present invention. Related person can clearly realize and apply the techniques disclosed herein by making some changes, appropriate alterations or combinations to the methods without departing from spirit, principles and scope of the present disclosure.

In order to further understand the invention, it is detailed below through examples.

### Example

Unless otherwise specified, "the compound of Formula (I)" used in the following examples refers to the solid compound prepared according to the method described in Example 10 of the patent application WO2018188590A1.

### Example 1: Preparation and Characterization of Crystal Form I

### 1. Preparation of Crystal Form I

### Method 1:

The compound of Formula (I) (51 mg) was weighed and added to a mixed solvent of dimethyl sulfoxide (0.15 mL) and n-propyl acetate (1.5 mL), and heated to 102°C to dissolve. The mixture was maintained at the temperature with stirring for 30 minutes, then allowed to cool naturally to room temperature after turning off the heating, and continued stirring for crystallization for 4 hours. The resulting mixture was filtered under suction, and the filter cake was washed with n-propyl acetate (1.0 mL × 2), and dried under vacuum at room temperature for 4 hours to afford a white solid (56 mg, 109.80%).

### Method 2:

The compound of Formula (I) (52 mg) was weighed and added to a mixed solvent of dimethyl sulfoxide (0.15 mL) and isobutyl acetate (1.5 mL), and heated to 116°C to dissolve. The mixture was maintained at the temperature with stirring for 30 minutes, then allowed to cool naturally to room temperature, and continued stirring for crystallization for 4 hours. The resulting mixture was filtered under suction, and the filter cake was washed with isobutyl acetate (1.0 mL × 2) and dried under vacuum at room temperature for 5 hours to afford a white solid (39 mg, 75.00%).

### 2. Characterization of Crystal Form I

(1) Characterization by Empyrean X-ray powder diffraction (XRPD) analysis: Using Cu-Kα radiation, exhibiting characteristic peaks at 2θ angles of: 6.73°, 9.98°, 10.93°, 11.48°, 12.24°, 13.40°, 13.67°, 14.29°, 15.31°, 15.81°, 16.34°, 16.82°, 17.10°, 17.73°, 18.13°, 18.72°, 19.96°, 20.32°, 20.95°, 21.85°, 22.32°, 23.03°, 23.62°, 24.16°, 24.53°, 25.25°, 25.63°, 26.83°, 27.30°, 28.06°, 29.09°, 29.82°, 30.10°, 30.46°, 30.79°, 31.70°, 32.68°, 33.25°, 33.82°, 34.32°, 35.39°, 36.27°, 36.65°, 37.14°, 37.93°, 38.80°, 40.83°, 41.47°, 42.42°, 43.60°, 45.00°, 46.76°, and 47.63°, with an allowable error margin of ±0.2°; the X-ray powder diffraction pattern is substantially as shown in Figure 1.
(2) Characterization by TA Q2000 differential scanning calorimetry (DSC) analysis: With a scanning rate of 10°C/min, showing endothermic peaks at 159.14°C and 268.85°C, with an allowable error margin of ±3°C.
(3) Characterization by TA Q500 thermogravimetric analysis (TGA): With a heating rate of 10°C/min, showing a weight loss of 26.56%, with an allowable error margin of ±0.1%.

### Example 2: Preparation and Characterization of Crystal Form II

### 1. Preparation of Crystal Form II

Method 1: Crystal Form I or Crystal Form IX (510 mg) was weighed and added to acetone (30.0 mL) and slurried at 60°C for 8 hours. Heating was stopped, and the reaction was allowed to cool naturally to room temperature. The mixture was filtered under suction, and the filter cake was washed with acetone (2.0 mL × 2) and dried under vacuum at 45°C for 12 hours to afford a white solid (380 mg, 74.51%).

Method 2: The compound of Formula (I) (101 mg) was weighed and added to N,N-dimethylformamide (0.3 mL) and heated to 77°C to dissolve the solid. After maintaining at the temperature for 10 minutes, ethyl acetate (3.0 mL) was slowly added dropwise, resulting in the precipitation of a white solid. The mixture was kept at the temperature for an additional 30 minutes, then allowed to cool naturally to room temperature after stopping heating. The mixture was filtered under suction, and the filter cake was washed with ethyl acetate (1.0 mL × 2), and dried under vacuum at 60°C overnight to afford a white solid (59 mg, 58.42%).

Method 3: The compound of Formula (I) (1.00 g) was weighed and added to a mixed solvent of dimethyl sulfoxide (2.5 mL) and sec-butanol (2.0 mL) and heated to 90°C to dissolve the solid. Sec-butanol (14.0 mL) was then slowly added dropwise. After complete addition, the mixture was maintained at the temperature for 30 minutes, then allowed to cool naturally to room temperature after stopping heating. The mixture was filtered under suction, and the filter cake was washed with sec-butanol (1.0 mL × 3), and dried under vacuum at 60°C overnight to afford a white solid (0.68 g, 68.00%).

Method 4: The compound of Formula (I) (70.03 g) was weighed and added to a mixed solvent of dimethyl sulfoxide (175.0 mL) and n-butanol (140.0 mL) and heated to 90°C to dissolve the solid. n-Butanol (980.0 mL) was then slowly added dropwise. After complete addition, the mixture was stirred at the temperature for 30 minutes, then cooled to room temperature at a rate of 5°C per 20 minutes, followed by additional stirring for 1 hour. The mixture was filtered under suction, and the filter cake was washed with n-butanol (50.0 mL × 3), and dried under vacuum at 60°C overnight to afford a white solid (45.48 g, 64.94%).

### 2. Characterization of Crystal Form II

(1) Characterization by Empyrean X-ray powder diffraction (XRPD) analysis: Using Cu-Kα radiation, exhibiting characteristic peaks at 2θ angles of: 6.10°, 7.25°, 8.75°, 10.50°, 11.92°, 12.24°, 14.67°, 15.32°, 16.43°, 18.37°, 18.84°, 20.06°, 20.73°, 21.03°, 21.86°, 23.49°, 24.00°, 25.00°, 25.79°, 26.03°, 26.49°, 26.93°, 27.44°, 28.68°, 29.63°, 30.98°, 33.41°, 35.13°, 35.56°, 36.35°, 37.26°, 38.77°, 39.62°, 40.98°, 41.69°, 44.53°, 48.16°, 51.26°, 52.76°, 57.61° and 58.92°, with an allowable error margin of ±0.2°; the X-ray powder diffraction pattern is substantially as shown in Figure 11.
(2) Characterization by TA Q2000 differential scanning calorimetry (DSC) analysis: With a scanning rate of 10°C/min, showing an endothermic peak at 269.71°C, with an allowable error margin of ±3°C; the differential scanning calorimetry thermogram is substantially as shown in Figure 12.
(3) Characterization by TA Q500 thermogravimetric analysis (TGA): With a heating rate of 10°C/min, showing a weight loss of 0.12%, with an allowable error margin of ±0.1%; the thermogravimetric analysis profile is substantially as shown in Figure 13.

### Example 3: Preparation and Characterization of Crystal Form III

### 1. Preparation of Crystal Form III

Method 1: The compound of Formula (I) (520 mg) was weighed and added to dimethyl sulfoxide (10.0 mL) and stirred at room temperature until dissolved. Water (10.0 mL) was slowly added dropwise, resulting in precipitation. After complete addition, stirring was continued for 4 hours. The mixture was filtered under suction, and the filter cake was washed with water (2.0 mL × 2), and dried under vacuum at 55°C for 15 hours to afford a pale yellow solid (380 mg, 73.08%).

Method 2: The compound of Formula (I) (50 mg) was weighed and added to N,N-dimethylformamide (2.0 mL) and stirred at room temperature until dissolved. Water (4.0 mL) was slowly added dropwise, resulting in precipitation. After complete addition, stirring was continued for 3 hours. The mixture was filtered under suction, and the filter cake was washed with water (0.5 mL × 2), and dried under vacuum at 50°C for 4 hours to afford a pale yellow solid (35 mg, 70.00%).

Method 3: The compound of Formula (I) (50 mg) was weighed and added to N,N-dimethylacetamide (2.0 mL) and stirred at room temperature until dissolved. Water (4.0 mL) was slowly added dropwise, resulting in precipitation. After complete addition, stirring was continued for 3 hours. The mixture was filtered under suction, and the filter cake was washed with water (0.5 mL × 2), and dried under vacuum at room temperature overnight to afford a pale yellow solid (13 mg, 26.00%).

### 2. Characterization of Crystal Form III

(1) Characterization by Empyrean X-ray powder diffraction (XRPD) analysis: Using Cu-Kα radiation, exhibiting characteristic peaks at 2θ angles of: 5.72°, 6.11°, 6.88°, 8.82°, 9.49°, 11.89°, 12.20°, 12.46°, 13.05°, 13.71°, 14.82°, 15.51°, 16.91°, 17.19°, 17.67°, 18.76°, 19.63°, 20.08°, 20.50°, 20.89°, 22.03°, 23.99°, 25.21°, 26.63°, 27.72°, 29.82°, 31.44°, 34.65°, 35.87°, 37.89°, 40.89° and 42.40°, with an allowable error margin of ±0.2°; the X-ray powder diffraction pattern is substantially as shown in Figure 2.
(2) Characterization by TA Q2000 differential scanning calorimetry (DSC) analysis: With a scanning rate of 10°C/min, showing an endothermic peak at 259.12°C, with an allowable error margin of ±3°C.
(3) Characterization by TA Q500 thermogravimetric analysis (TGA): With a heating rate of 10°C/min, showing a weight loss of 0.58%, with an allowable error margin of ±0.1%.

### Example 4: Preparation and Characterization of Crystal Form IV

### 1. Preparation of Crystal Form IV

Method 1: Crystal Form I or Crystal Form IX (50 mg) was weighed and added to tetrahydrofuran (4.0 mL) and slurried under reflux for 4.5 hours. The mixture was allowed to cool naturally to room temperature after stopping heating, and filtered under suction, and the filter cake was washed with tetrahydrofuran (0.5 mL × 2) and dried under vacuum at 60°C for 36 hours to afford a white solid (32 mg, 64.00%).

Method 2: Crystal Form I or Crystal Form IX (50 mg) was weighed and added to 1,4-dioxane (3.0 mL) and heated to 85°C to dissolve. The mixture was maintained at the temperature with stirring for 30 minutes, then allowed to cool naturally to room temperature after stopping heating and stirred overnight. The resulting mixture was filtered under suction, and the filter cake was washed with 1,4-dioxane (0.5 mL × 2), and dried under vacuum at room temperature for 5 hours to afford a pale yellow solid (27 mg, 54.00%).

Method 3: Crystal Form I or Crystal Form IX (49 mg) was weighed and added to a mixed solvent of acetic acid (0.1 mL) and tetrahydrofuran (1.5 mL) and stirred at room temperature until dissolved. After 2 hours, a white solid was gradually precipitated. Stirring was continued for an additional 5 hours to complete crystallization. The mixture was filtered under suction, and the filter cake was washed with tetrahydrofuran (0.5 mL × 2), and dried under vacuum at room temperature for 6 hours to afford a white solid (20 mg, 40.82%).

### 2. Characterization of Crystal Form IV

(1) Characterization by Empyrean X-ray powder diffraction (XRPD) analysis: Using Cu-Kα radiation, exhibiting characteristic peaks at 2θ angles of:6.96°, 7.63°, 11.62°, 12.85°, 13.38°, 13.76°, 13.91°, 14.89°, 16.13°, 16.59°, 17.85°, 19.00°, 19.23°, 19.69°, 20.88°, 21.59°, 21.89°, 23.10°, 23.33°, 24.06°, 24.57°, 25.16°, 26.00°, 26.50°, 27.61°, 28.01°, 28.31°, 29.94°, 30.72°, 31.53°, 32.25°, 32.75°, 33.06°, 34.99°, 36.14°, 36.99°, 38.65°, 40.19°, 42.15°, 45.98°, 47.18°, 47.69°, 48.37°, 49.42°, 50.43°, 51.62°, 54.04° and 54.63°, with an allowable error margin of ±0.2°; the X-ray powder diffraction pattern is substantially as shown in Figure 3.
(2) Characterization by TA Q2000 differential scanning calorimetry (DSC) analysis: With a scanning rate of 10°C/min, showing an endothermic peak at 211.51°C, with an allowable error margin of ±3°C.
(3) Characterization by TA Q500 thermogravimetric analysis (TGA): With a heating rate of 10°C/min, showing a weight loss of 3.11%, with an allowable error margin of ±0.1%.

### Example 5: Preparation and Characterization of Crystal Form V

### 1. Preparation of Crystal Form V

Crystal Form I or Crystal Form IX (0.51 g) was weighed and added to dichloromethane (50.0 mL) and slurried at 40°C for 5 hours. The mixture was allowed to cool naturally to room temperature after stopping heating, filtered under suction, and the filter cake was washed with dichloromethane (2.0 mL × 2) and dried under vacuum at 55°C for 12 hours to afford a pale yellow solid (0.31 g, 60.78%).

### 2. Characterization of Crystal Form V

(1) Characterization by Empyrean X-ray powder diffraction (XRPD) analysis: Using Cu-Kα radiation, exhibiting characteristic peaks at 2θ angles of:7.24°, 8.02°, 11.95°, 13.47°, 14.19°, 15.03°, 17.35°, 18.31°, 19.38°, 19.86°, 20.30°, 22.02°, 22.98°, 23.88°, 24.05°, 25.19°, 25.61°, 26.75°, 28.03°, 28.83°, 30.22°, 30.87°, 31.46°, 32.28°, 33.40°, 34.19°, 35.66°, 36.91°, 37.47°, 38.21°, 39.28°, 43.05°, 48.83°, 49.45° and 51.38°, with an allowable error margin of ±0.2°; the X-ray powder diffraction pattern is substantially as shown in Figure 4.
(2) Characterization by TA Q2000 differential scanning calorimetry (DSC) analysis: With a scanning rate of 10°C/min, showing endothermic peaks at 214.12°C and 268.62°C, with an allowable error margin of ±3°C.
(3) Characterization by TA Q500 thermogravimetric analysis (TGA): With a heating rate of 10°C/min, showing a weight loss of 0.28%, with an allowable error margin of ±0.1%.

### Example 6: Preparation and Characterization of Crystal Form VI

### 1. Preparation of Crystal Form VI

The compound of Formula (I) (0.41 g) was weighed and added to formic acid (4.0 mL) and stirred at room temperature until dissolved. Methyl tert-butyl ether (16.0 mL) was then slowly added dropwise, resulting in precipitation of a white solid. Crystallization was continued with stirring for 3 hours. The mixture was filtered under suction, and the filter cake was washed with methyl tert-butyl ether (3.0 mL × 2), and dried under vacuum at 50°C for 20 hours to afford a white solid (0.35 g, 85.37%).

### 2. Characterization of Crystal Form VI

(1) Characterization by Empyrean X-ray powder diffraction (XRPD) analysis: Using Cu-Kα radiation, exhibiting characteristic peaks at 2θ angles of:4.25°, 7.97°, 8.29°, 9.83°, 12.71°, 15.72°, 16.95°, 17.39°, 18.39°, 19.54°, 20.19°, 21.20°, 22.84°, 23.66°, 24.11°, 25.23°, 26.18°, 27.13°, 27.79°, 28.17°, 31.40°, 32.50° and 34.83°, with an allowable error margin of ±0.2°; the X-ray powder diffraction pattern is substantially as shown in Figure 5.
(2) Characterization by TA Q2000 differential scanning calorimetry (DSC) analysis: With a scanning rate of 10°C/min, showing endothermic peaks at 184.88°C and 247.61°C, with an allowable error margin of ±3°C.
(3) Characterization by TA Q500 thermogravimetric analysis (TGA): With a heating rate of 10°C/min, showing a weight loss of 9.67%, with an allowable error margin of ±0.1%.

### Example 7: Preparation and Characterization of Crystal Form VII

### 1. Preparation of Crystal Form VII

The compound of Formula (I) (51 mg) was weighed and added to ethylene glycol (5.0 mL) and heated to 90°C to dissolve. The mixture was maintained at the temperature with stirring for 30 minutes, then allowed to cool naturally after stopping heating. The mixture was stirred at room temperature for 60 hours with solid precipitation. The mixture was filtered under suction, and the filter caked was washed with ethylene glycol (0.5 mL × 2), and dried under vacuum at room temperature for 8 hours to afford a yellow solid (16 mg, 31.37%).

### 2. Characterization of Crystal Form VII

(1) Characterization by Empyrean X-ray powder diffraction (XRPD) analysis: Using Cu-Kα radiation, exhibiting characteristic peaks at 2θ angles of:6.78°, 7.04°, 8.53°, 9.14°, 9.80°, 9.98°, 10.75°, 12.28°, 13.14°, 13.53°, 14.21°, 16.93°, 17.35°, 17.71°, 18.05°, 18.71°, 19.58°, 21.06°, 21.51°, 23.38°, 23.85°, 24.71°, 25.17°, 26.10°, 26.38°, 26.82°, 28.15°, 28.48°, 30.48°, 31.94°, 33.99°, 34.80°, 35.92°, 36.89°, 40.06°, 41.58°, 47.64° and 51.33°, with an allowable error margin of ±0.2°; the X-ray powder diffraction pattern is substantially as shown in Figure 6.
(2) Characterization by TA Q2000 differential scanning calorimetry (DSC) analysis: With a scanning rate of 10°C/min, showing endothermic peaks at 95.38°C, 172.09°C and 254.53°C, with an allowable error margin of ±3°C.
(3) Characterization by TA Q500 thermogravimetric analysis (TGA): With a heating rate of 10°C/min, showing a weight loss of 19.65%, with an allowable error margin of ±0.1%.

### Example 8: Preparation and Characterization of Crystal Form VIII

### 1. Preparation of Crystal Form VIII

Method 1: The compound of Formula (I) (200 mg) was weighed and added to a mixed solvent of formamide (2.0 mL) and acetone (2.0 mL), heated to 55°C to dissolve, and maintained at the temperature with stirring for 30 minutes. After stopping heating and cooling naturally to room temperature, a white solid precipitated. Crystallization was continued with stirring for 4 hours. The mixture was filtered under suction, and the filter caked was washed with acetone (1.0 mL × 2) and dried under vacuum at room temperature for 17 hours to afford a white solid (82 mg, 41.00%).

Method 2: The compound of Formula (I) (503 mg) was weighed and added to a mixed solvent of formamide (5.0 mL) and ethyl acetate (5.0 mL), heated to 77°C to dissolve the solid. The mixture was allowed to cool naturally to room temperature after stopping heating and further stirred overnight, resulting in the precipitation of a white solid. The mixture was filtered under suction, and the filter caked was washed with ethyl acetate (1.0 mL × 3), and dried under vacuum at 60°C for 8 hours to afford a white solid (312 mg, 62.03%).

Method 3: The compound of Formula (I) (502 mg) was weighed and added to formamide (10.0 mL), heated to 110°C to dissolve the solid. After stopping heating, the mixture was allowed to cool naturally to room temperature and a white solid precipitated. The mixture was filtered under suction, and the filter caked was washed with ethyl acetate (3.0 mL × 3), and dried under vacuum at 60°C overnight to afford a white solid (313 mg, 62.35%).

### 2. Characterization of Crystal Form VIII

(1) Characterization by Empyrean X-ray powder diffraction (XRPD) analysis: Using Cu-Kα radiation, exhibiting characteristic peaks at 2θ angles of:5.64°, 7.47°, 9.64°, 10.03°, 12.36°, 14.65°, 15.50°, 16.66°, 17.01°, 17.68°, 18.27°, 19.16°, 20.13°, 22.05°, 22.64°, 23.84°, 24.15°, 25.41°, 26.50°, 27.27°, 27.96°, 29.45°, 29.98°, 31.21°, 32.19°, 33.62°, 34.31°, 35.20°, 36.76°, 37.45°, 38.74°, 39.33°, 39.95°, 42.01°, 44.55°, 46.94°, 48.39° and 51.09°, with an allowable error margin of ±0.2°; the X-ray powder diffraction pattern is substantially as shown in Figure 7.
(2) Characterization by TA Q2000 differential scanning calorimetry (DSC) analysis: With a scanning rate of 10°C/min, showing endothermic peaks at 140.23°C, 171.86°C and 259.47°C, with an allowable error margin of ±3°C.
(3) Characterization by TA Q500 thermogravimetric analysis (TGA): With a heating rate of 10°C/min, showing a weight loss of 18.24%, with an allowable error margin of ±0.1%.

### Example 9: Preparation and Characterization of Crystal Form IX

### 1. Preparation of Crystal Form IX

The compound of Formula (I) (50 mg) was weighed and added to a mixed solvent of dimethyl sulfoxide (0.2 mL) and ethyl acetate (4.0 mL), heated to 80°C to dissolve the solid, and maintained the temperature with stirring for 30 minutes. After stopping heating, the mixture was allowed to cool naturally to room temperature and stirred for crystallization for 4 hours. The resulting mixture was filtered under suction, and the filter cake was washed with ethyl acetate (0.5 mL × 2), and dried under vacuum at room temperature for 6 hours to afford a white solid (40 mg, 80.00%).

### 2. Characterization of Crystal Form IX

(1) Characterization by Empyrean X-ray powder diffraction (XRPD) analysis: Using Cu-Kα radiation, exhibiting characteristic peaks at 2θ angles of:7.49°, 9.32°, 9.84°, 11.18°, 11.39°, 12.63°, 13.45°, 15.04°, 15.94°, 16.30°, 16.86°, 17.87°, 18.54°, 18.68°, 18.84°, 19.09°, 19.74°, 19.97°, 20.09°, 21.02°, 22.19°, 22.44°, 22.88°, 23.21°, 23.53°, 24.46°, 25.35°, 25.58°, 25.93°, 26.34°, 26.65°, 26.98°, 27.56°, 27.93°, 28.15°, 28.76°, 29.00°, 29.71°, 30.19°, 30.79°, 31.20°, 32.25°, 32.86°, 33.33°, 33.63°, 34.04°, 34.64°, 35.35°, 35.98°, 36.63°, 37.73°, 38.67°, 39.54°, 40.11°, 41.28°, 42.78°, 43.54°, 44.32°, 44.95°, 46.00°, 48.05°, 48.48°, 49.24°, 50.09° and 58.19°, with an allowable error margin of ±0.2°; the X-ray powder diffraction pattern is substantially as shown in Figure 8.
(2) Characterization by TA Q2000 differential scanning calorimetry (DSC) analysis: With a scanning rate of 10°C/min, showing endothermic peaks at 161.94°C and 268.77°C, with an allowable error margin of ±3°C.
(3) Characterization by TA Q500 thermogravimetric analysis (TGA): With a heating rate of 10°C/min, showing a weight loss of 26.81%, with an allowable error margin of ±0.1%.

### Example 10: Preparation and Characterization of Crystal Form X

### 1. Preparation of Crystal Form X

Method 1: The compound of formula (I) (501 mg) was weighed and added to formic acid (5.0 mL), and stirred at room temperature until the solid was dissolved. Ethyl formate (200.0 mL) was slowly added dropwise, resulting in gradual precipitation of a white solid. The mixture was continued stirring for 4 hours, then filter under suction. The filter cake was washed with ethyl formate (5.0 mL × 2) and dried under vacuum at room temperature overnight to obtain a white solid (333 mg, 66.47%).

Method 2: The compound of formurla (I) (1.00 g) was weighed and added to a mixed solvent of formic acid (2.0 mL) and acetone (10.0 mL). The mixture was heated to 55°C until the solid dissolved, then acetone (20.0 mL) was slowly added dropwise, resulting in gradual precipitation of a white solid. The mixture was maintained at the temperature and stirred for 30 minutes, then allowed to cool naturally to room temperature after turn off the heating. The mixture was filtered under suction, the filter cake was washed with acetone (1.0 mL × 3), and dried under vacuum at room temperature overnight to obtain a white solid (542 mg, 54.20%).

### 2. Characterization of Crystal Form X

(1) Characterization by Empyrean X-ray powder diffraction (XRPD) analysis: Using Cu-Kα radiation, exhibiting characteristic peaks at 2θ angles of:5.63°, 7.04°, 9.98°, 10.37°, 11.48°, 13.12°, 15.17°, 16.00°, 17.19°, 17.73°, 18.76°, 19.91°, 20.64°, 21.65°, 22.88°, 25.04°, 25.55°, 26.35°, 27.42°, 28.67°, 30.16°, 32.02°, 32.54°, 33.87°, 35.17°, 36.15°, 37.90°, 40.31°, 41.90°, 42.65°, 43.83°, 45.72°, 46.56°, 51.62°, 52.35° and 53.12°, with an allowable error margin of ±0.2°; the X-ray powder diffraction pattern is substantially as shown in Figure 9.
(2) Characterization by TA Q2000 differential scanning calorimetry (DSC) analysis: With a scanning rate of 10°C/min, showing endothermic peaks at 175.16°C and 252.36°C, with an allowable error margin of ±3°C.
(3) Characterization by TA Q500 thermogravimetric analysis (TGA): With a heating rate of 10°C/min, showing a weight loss of 17.14%, with an allowable error margin of ±0.1%.

### Example 11: Preparation and Characterization of Amorphous Form

### 1. Preparation of Amorphous Form

The compound of formula (I) (60 mg) was weighed and ground in a mortar for 1.5 hours, then collecting a white powder (15 mg, 25.00%).

### 2. Characterization of Amorphous Form

The amorphous form was characterized by Empyrean X-ray powder diffraction (XRPD) analysis using Cu-Kα radiation. The obtained XRPD pattern is substantially as shown in Figure 10.

### Example 12 Stability Studies

(1) High-Temperature Test:
   Place an appropriate amount of the test sample in a flat weighing bottle, spreading it into a thin layer ≤5 mm thick. Store at 60°C ± 2°C for 30 days. Collect samples at 0, 5, 10, and 30 days for analysis according to the key stability test items. Observe any color changes and determine purity by HPLC.
(2) High-Humidity Test:
   Place an appropriate amount of the test sample from one batch in a flat weighing bottle, spreading it into a thin layer ≤5 mm thick. Store at 25°C and 90% ± 5% relative humidity (RH) for 30 days. Collect samples at 0, 5, 10, and 30 days for analysis according to the key stability test items. Observe any color changes and determine purity by HPLC.
(3) Light-Exposure Test:
   Expose the sample to visible light (4500 lx ± 500 lx) and UV light (energy ≥0.7 W·h/m²) for 30 days. Collect samples at 0, 5, 10, 15, and 30 days for analysis according to the key stability test items. Observe any color changes and determine purity by HPLC.
(4) Accelerated Test:
   Package an appropriate amount of the test sample from one batch in single-layer PE with aluminum foil. Store at 40°C ± 2°C / 75% ± 5% RH for 3 months. Collect samples at 0, 1, 2, 3, and 6 months for analysis according to the key stability test items. Observe any color changes and determine purity by HPLC.

Results were as shown in table 1 and table 2.

**Table 1 Results of High-Temperature, High-Humidity, and Light-Exposure Tests for the Crystal Form of the Present Invention**

| Test Results of Crystal Form II under High-Temperature, High-Humidity, and Light-Exposure Conditions | | | | |
|---|---|---|---|---|
| Conditions | | High-Temperature | High-Humidity | Light-Exposure |
| Time | 0 day | 30 day | 30 day | 30 day |
| Outward | Off-white | Off-white | Off-white | Pale yellow |
| Purity (%) | 99.97 | 99.93 | 99.96 | 99.92 |

| Test Results of Crystal Form V under High-Temperature, High-Humidity, and Light-Exposure Conditions | | | | |
|---|---|---|---|---|
| Conditions | | High-Temperature | High-Humidity | Light-Exposure |
| Time | 0 day | 30 day | 30 day | 30 day |
| Outward | Off-white | Off-white | Off-white | Pale yellow |
| Purity (%) | 99.52 | 99.47 | 99.51 | 99.36 |

**Table 2 Results of Accelerated Test for the Crystal Form II of the Present Invention**

| Time | 0 day | 1 month | 2 month | 3 month | 6 month |
|---|---|---|---|---|---|
| Outward | Off-white | Off-white | Off-white | Off-white | Off-white |
| Purity (%) | 99.96 | 99.96 | 99.95 | 99.98 | 99.93 |

### Conclusion:

Experimental results demonstrate that Crystal Form II of the present invention exhibits excellent stability under various storage conditions, making it suitable for pharmaceutical uses.

### Example 13 The hygroscopicity test

An appropriate amount of the test sample was taken, and its hygroscopicity was measured using a dynamic vapor sorption (DVS) instrument. The experimental results are shown in Table 4. The classification criteria for hygroscopicity characteristics and moisture absorption weight gain (as per Appendix 9103 "Guidelines for Hygroscopicity Testing of Drugs" from the Chinese Pharmacopoeia 2020 Edition, test conditions: 25°C ± 1°C, 80% ± 2% relative humidity (RH)) are described in Table 3 below:

**Table 3 The description of the hygroscopicity feature and the definition of the hygroscopicity gain**

| | |
|---|---|
| the hygroscopicity feature | the hygroscopicity gain |
| deliquescence | Absorb enough water to form a liquid |
| highly hygroscopicity | Not less than 15% |
| hygroscopicity | less than 15% but not less than 2% |
| lightly hygroscopicity | less than 2% but not less than 0.2% |
| No or almost none hygroscopicity | less than 0.2% |

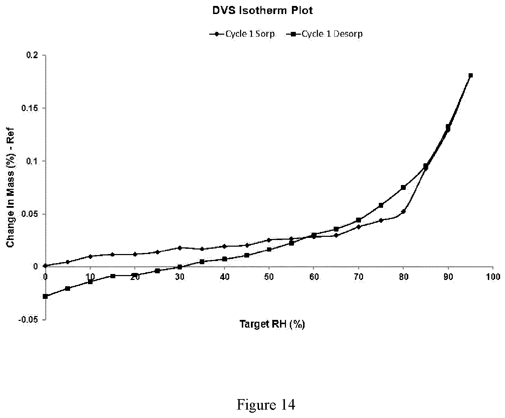

The hygroscopicity test results for Crystal Form II of the present invention are shown in Table 4, with the corresponding DVS profile substantially as presented in Figure 14. The experimental results demonstrate that Crystal Form II of the present invention exhibits no or negligible hygroscopicity and is not prone to deliquescence under high humidity conditions.

**Table 4 Hygroscopicity Test Results**

| Test sample | Weight Gain at 60% RH (%) | Weight Gain at 80% RH (%) | Weight Gain at 95% RH (%) |
|---|---|---|---|
| Crystal Form II | 0.02 | 0.05 | 0.18 |

### Example 14 The pharmacokinetics test

Male Beagle dogs of 8-12 kg were divided into 3 groups, 3 dogs in each group, and capsules containing test samples were orally administered at a dose of 5 mg/kg, and blood was collected at time points of 0.25, 0.5, 1.0, 2.0, 4.0, 6.0, 8.0 and 24 h. Standard curve was plotted based on concentrations of the samples in a suitable range, the concentration of the test sample in the plasma sample was measured and quantified by AB SCIEX API4000-type LC-MS / MS at MRM mode. Pharmacokinetic parameters were calculated according to drug concentration -time curve using a noncompartmental method by WinNonLin 6.3 software. The experimental results demonstrate that the crystal form of the present invention exhibits excellent pharmacokinetic properties.

The foregoing description is merely a basic illustration of the present invention and any equivalent transformation made in accordance with the technical solution of the present invention is intended to be within the scope of the present invention.

Reference throughout this specification to "an embodiment," "some embodiments," "one embodiment", "another example," "an example," "a specific examples," or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example, "in an example," "in a specific examples," or "in some examples," in various places throughout this specification are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A crystal form of the compound represented by Formula (I), which is Crystal Form II: 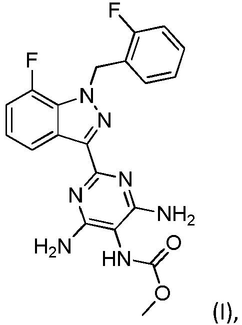 **characterized in that** the X-ray powder diffraction pattern of the Crystal Form II has characteristic peaks at the following 2θ angles: 7.25°, 11.92°, 12.24°, 15.32° and 20.73°, wherein the error range of each peak is ±0.2°.

2. The crystal form according to claim 1, **characterized in that** the X-ray powder diffraction pattern of the Crystal Form II further has characteristic peaks at at least one of the following 2θ angles: 6.10°, 8.75°, 14.67°, 16.43° and 29.63°, wherein the error range of each peak is ±0.2°.

3. The crystal form according to claim 1 or 2, **characterized in that** the X-ray powder diffraction pattern of the Crystal Form II further has characteristic peaks at at least one of the following 20 angles: 18.37°, 18.84°, 20.06°, 24.00°, 25.79°, 26.03°, 27.44°, 30.98°, 33.41°, 35.13°, 37.26°, 38.77°, 40.98° and 44.53°, wherein the error range of each peak is ±0.2°.

4. The crystal form according to any one of claims 1 to 3, **characterized in that** the X-ray powder diffraction pattern of the Crystal Form II further has characteristic peaks at at least one of the following 2θ angles: 10.50°, 21.03°, 21.86°, 23.49°, 25.00°, 26.49°, 26.93°, 28.68°, 33.41°, 35.56°, 36.35°, 39.62° and 48.16°, wherein the error range of each peak is ±0.2°.

5. The crystal form according to any one of claims 1 to 4, **characterized in that** the Crystal Form II has an X-ray powder diffraction pattern substantially as shown in Figure 11.

6. The crystal form according to any one of claims 1 to 5, **characterized in that** the differential scanning calorimetry thermogram of the Crystal Form II comprises an endothermic peak at 269.71°C ± 3°C.

7. The crystal form according to any one of claims 1 to 6, **characterized in that** the Crystal Form II has a differential scanning calorimetry thermogram substantially as shown in Figure 12.

8. A pharmaceutical composition comprising the crystal form according to any one of claims 1-7 and a pharmaceutically acceptable excipient.

9. The pharmaceutical composition according to claim 8, wherein the excipient is at least one of a carrier, an adjuvant, a diluent, and a vehicle.

10. Use of the crystal form according to any one of claims 1-7 or the pharmaceutical composition according to claim 8 or 9 in the manufacture of a medicament, wherein the medicament is used for treating and/or preventing heart failure, angina pectoris, hypertension, pulmonary hypertension, ischemia, vascular disease, kidney diseases, thromboembolic disease, male sexual dysfunction, systemic sclerosis, sickle cell anemia, achalasia, fibrotic disease, and/or atherosclerosis.

11. The crystal form according to any one of claims 1-7 or the pharmaceutical composition according to claim 8 or 9 for use in the treatment and/or prevention of heart failure, angina pectoris, hypertension, pulmonary hypertension, ischemia, vascular disease, kidney diseases, thromboembolic disease, male sexual dysfunction, systemic sclerosis, sickle cell anemia, achalasia, fibrotic disease, and/or atherosclerosis.

12. A method for treating, alleviating and/or preventing a disease, wherein the disease is heart failure, angina pectoris, hypertension, pulmonary hypertension, ischemia, vascular disease, kidney diseases, thromboembolic disease, male sexual dysfunction, systemic sclerosis, sickle cell anemia, achalasia, fibrotic disease and/or atherosclerosis, and the method comprises administering an effective amount of the crystal form according to any one of claims 1-7 or the pharmaceutical composition according to claim 8 or 9.
